(19) Europäisches Patentamt
European Patent Office
Office européen des brevets

(11) EP 1 505 051 A1

(12) EUROPÄISCHE PATENTANMELDUNG

(43) Veröffentlichungstag:
**09.02.2005 Patentblatt 2005/06**

(51) Int Cl.⁷: **C07C 49/403**, C07C 45/62

(21) Anmeldenummer: **04014734.0**

(22) Anmeldetag: **23.06.2004**

(84) Benannte Vertragsstaaten:
**AT BE BG CH CY CZ DE DK EE ES FI FR GB GR HU IE IT LI LU MC NL PL PT RO SE SI SK TR**
Benannte Erstreckungsstaaten:
**AL HR LT LV MK**

(30) Priorität: **01.08.2003 DE 10335248**

(71) Anmelder: **Degussa AG**
**40474 Düsseldorf (DE)**

(72) Erfinder:
- **Kleinfeld, Susanne, Dr.**
  **63538 Grosskrotzenburg (DE)**
- **Fischer, Achim, Dr.**
  **63739 Aschaffenburg (DE)**
- **Wilz, Frank**
  **63755 Alzenau (DE)**
- **Weckbecker, Christoph, Dr.**
  **63584 Gründau-Lieblos (DE)**
- **Huthmacher, Klaus, Dr.**
  **63571 Gelnhausen (DE)**

(54) **Verfahren zur Herstellung von 2,2,6-Trimethyl-cyclohexan-1-on**

(57) Die vorliegende Erfindung betrifft ein Verfahren zur Herstellung von 2,2,6-Trimethyl-cyclohexan-1-on (TMCH) durch partielle Reduktion von 2,6,6-Trimethyl-2-cyclohexan-1,4-dion (Ketoisophoron, KIP), bei dem man die Reaktion in Gegenwart oxidischer Katalysatoren in der Gasphase durchführt.

EP 1 505 051 A1

## Beschreibung

Die vorliegende Erfindung betrifft ein Verfahren zur Herstellung von 2,2,6-Trimethyl-cyclohexan-1-on (TMCH) durch Reduktion von 2,6,6-Trimethyl-2-cyclohexan-1,4-dion (Ketoisophoron, KIP) in der Gasphase.

Stand der Technik

TMCH ist ein wertvolles Zwischenprodukt für zahlreiche Riechstoffsynthesen. Neuere Carotenoid- und Vitamin A-Synthesen basieren auf TMCH. So stellt man den Riechstoff β-Damascon auf einfache Weise durch Additionsreaktion von 3-tert-Butyloxy-1-butin mit 2,2,6-Trimethyl-cyclohexan-1-on und anschließender saurer thermischer Behandlung her (DE 29 25 176 C2). In der US 5,808,120 wird die Umsetzung einer Gringnard-Verbindung von (Z) 3-Methyl-pent-2en-4ynol mit TMCH beschrieben, bei der man aus dem erhaltenen Produkt Retinol herstellt.
Wegen der wirtschaftlichen Bedeutung wurden verschiedene Wege vorgeschlagen, um zu der gewünschten Verbindung TMCH zu gelangen.
Dazu zählt ein mehrstufiges von Ketoisophoron ausgehendes Verfahren, wie es von Widmer, Pure and Applied Chemistry (1985) 57, 741-752, vorgeschlagen wird.

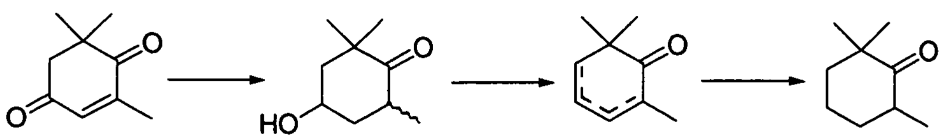

Die Bedingungen, unter denen die einzelnen Schritte ablaufen, werden in verschiedenen Dokumenten getrennt beschrieben:

1. Stereoselektive Reduktion von Ketoisophoron in zwei Stufen zum Ketoalkohol mit Raney-Nickel. (M. Soukup, Helv. Chim. Acta 1989, 72, 365-369)

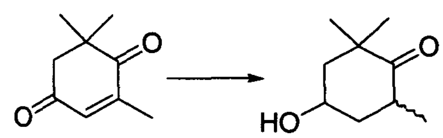

2. Wassereliminierung: Die Eliminierung von Ketoalkohol zu 2,2,6-Trimethyl-2-cyclohexenon bzw. Doppelbindungsisomeren ist nicht experimentell beschrieben. Es wird lediglich in dem oben angeführten Artikel von Widmer erwähnt, dass sie in Gegenwart von para-Toluolsulfonsäure abläuft:

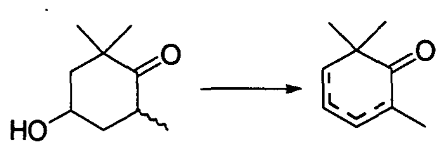

3. Die Reduktion von 2,2,6-Trimethyl-2-cyclohexenon zu TMCH wird zum Beispiel in der EP 00 32 287 B und der US 4,250,332 beschrieben:

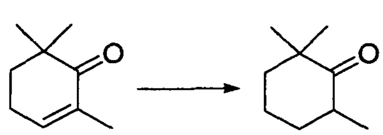

Bei M. von Arx et al. (J. Mol Catal. A.: Chemical (1999) 275-283) findet sich eine Übersicht über den Einfluss von Lösungsmitteln bei der Hydrierung von Ketoisophoron unter Verwendung von Raney-Nickel und Pt- und Pd $Al_2O_3$-Trägerkatalysatoren in der Flüssigphase. Es wird jedoch die Reduktion einer Ketogruppe und der Doppelbindung beschrieben.

Aufgabe der Erfindung

Da Ketoisophoron aus dem kostengünstigen α-Isophoron leicht in einem zweistufigen Verfahren zugänglich ist (EP 0 832 870 B, EP 0 832 871 B, DE 196 19 570 A),

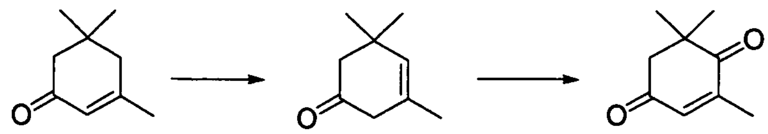

und sich daher als Vorstufe anbietet, ist es Aufgabe der Erfindung, ein einfaches Verfahren zur partiellen Reduktion von Ketoisophoron zu finden, das direkt zu TMCH führt.

Gegenstand der Erfindung

Gegenstand der Erfindung ist ein Verfahren zur Herstellung von 2,6,6-Trimethyl-2-cyclohexan-1-dion (TMCH) durch partielle Hydrierung von 2,6,6-Trimethyl-2-cyclohexen-1,4-dion unter Verwendung von heterogenen Katalysatoren, dadurch gekennzeichnet, dass man die Reaktion in der Gasphase durchführt und als Katalysatoren Oxide oder Mischoxide von Elementen, ausgewählt aus den Gruppen $I_a$, $II_a$, VIII, $I_b$, $II_b$, $III_a$, $IV_a$, $IV_b$, und $V_a$ des Periodensystems der Elemente einzeln oder im Gemisch einsetzt. Die Gruppeneinteilung der Haupt- und Nebengruppen .des Periodensystems der Elemente erfolgt nach der Bezeichnung gemäß IUPAC, Pure and Appl. Chem., 66, 2423-2444, 1994. So gehören zu Gruppe $II_a$ insbesondere Mg, Ca, Sr und Ba. Zur Gruppe VIII gehören Fe, Co, Ni, Ru, Rh, Pd, Os, Ir und Pt. Zu den Gruppen $I_b$, $III_a$ und $V_a$ zählen insbesondere die Elemente Cu, Ag, Au, B, Al, Ga, In, Sb und Bi, zur Gruppe $II_b$ Zn.

Unter Mischoxid wird eine Verbindung verstanden, in der Sauerstoff mit mehr als einem der aufgezählten Elemente eine Verbindung eingeht.

Aus der Gruppe $IV_a$ werden Si, Ge und Sn bevorzugt, aus der Gruppe $IV_b$ Ti und Zr.

Kohlenstoff aus der Gruppe $IV_a$ dient im allgemeinen zum Beispiel in Form von Aktivkohle als Träger, wenn es sich um edelmetallhaltige Katalysatoren handelt. Besonders bevorzugt sind dabei Pd und Pt, die auch in metallischer Form auf diesem oder anderen Träger vorhanden sein können, bevorzugt in Kombination mit weiteren oxidischen Verbindungen der Gruppen $II_a$, VIII und $I_b$. Letztere bevorzugt in einer Menge < 2 Gew.-%, bezogen auf den Katalysator.

Der Katalysator kann auch in geringen Mengen, bevorzugt > 0 bis < 5 %, insbesondere 0 bis < 1 Gew.-%, anionische Bestandteile wie Chlorid, Sulfat oder Phosphate enthalten.

Der bevorzugt als Trägerkatalysator eingesetzt Katalysator enthält die oxidischen Verbindungen im allgemeinen in einer Menge von jeweils > 0 bis 2 Gew.-%, bezogen auf den Katalysator.

Es sind zusätzlich zum gegebenenfalls oxidischen Trägermaterial wie z. B. $Al_2O_3$ oder $SiO_2$ mindestens zwei weitere Oxide oder Mischoxide in dem Katalysator zu finden.

Bevorzugt sind oxidische Verbindungen der Elemente, ausgewählt aus der Gruppe Na, K, Mg, Ca, Sr, Ba, Ti, Zr, Mo, Mn, Fe, Co, Ni, Cu, Zn, Al, Si oder Sn.

Als sehr geeignet hat sich die Verwendung von NiO enthaltenden Katalysatoren herausgestellt. Diese enthalten NiO in einer Menge von > 0,01 bis 50 Gew.-% und weitere oxidische Bestandteile.

Bevorzugt sind dabei die Oxide ausgewählt aus der Gruppe $TiO_2$, $SiO_2$, ZnO, CuO, $Fe_2O_3$ oder MgO.

Besonders bevorzugt sind Katalysatoren, die NiO, CuO und ZnO enthalten. Dabei liegt in besonders geeigneten Fällen entweder der NiO-Gehalt mit 5 bis 25 Gew.-% über der Gesamtmenge von CuO und ZnO mit jeweils > 0 bis 2 Gew.-%, oder der Gehalt von CuO und ZnO übertrifft mit insgesamt 5 bis 40 Gew.-% die NiO-Menge von > 0 bis 2 Gew.-%. In letzterem Fall enthält der Katalysator bevorzugt Pt (< 1 Gew.-%).

Die eingesetzten Katalysatoren sind in bekannter Weise erhältlich.

Sie können beispielsweise dadurch hergestellt werden, dass man als Ausgangsverbindungen der oxidischen Verbindungen geeignete Salze der sie konstituierenden elementaren Bestandteile, gegebenenfalls bei erhöhter Temperatur und unter Zusatz von Säuren oder Basen in wässrigen Medium durch Lösen oder Suspendieren fein verteilt,

mischt, anschließend den vorgesehenen Trägerkörper imprägniert und diesen dann in der Regel vortrocknet und bei 180 bis 480°C in inerter Atmosphäre calciniert.

Edelmetallverbindungen können durch reduzierende Reaktanden in die metallische Form überführt werden.

Die Form des Katalysators kann in Abhängigkeit von der Art des verwendeten Reaktors als Pulver, Tablette, Zylinder, Kugel etc. gewählt werden.

Vor der Benutzung wird der Katalysator vorteilhaft bei einer Temperatur von 200 bis 400°C, insbesondere 200 bis 280°C, durch ein reduzierendes Gas wie zum Beispiel Wasserstoff aktiviert.

Das Verfahren kann kontinuierlich oder batchweise betrieben werden, eine kontinuierliche Fahrweise ist jedoch bevorzugt.

Die Umsetzung des 2,6,6-Trimethyl-2-cyclohexan-1,4-dion mit Wasserstoff erfolgt in der Gasphase. Für die Wahl der Reaktionsparameter ist ein breiter Spielraum gegeben. In der Regel wird die Umsetzung drucklos oder unter Überdruck bis 10 bara bei Temperaturen zwischen 180 und 280°C, vorzugsweise bei Temperaturen zwischen 220 bis 270°C, durchgeführt. Es ist dabei vorteilhaft, das Reduktionsmittel als Wasserstoff zuzuführen. Das Mengenverhältnis von KIP und Wasserstoff kann in weiten Grenzen gewählt werden. Es ist jedoch zweckmässig, je Mol KIP etwa 1 bis 10 Mol Wasserstoff, vorzugsweise 1 bis 5 Mol Wasserstoff anzuwenden. Je Liter Schüttvolumen des Katalysators werden im allgemeinen bis zu 10 Mol KIP vorzugsweise 1 bis 5 Mol KIP eingespeist.

In einer kontinuierlichen Fahrweise können die nicht umgesetzten Reaktanten in einer Kreisgasfahrweise erneut auf den Reaktor gefahren werden. Dies bedeutet, dass auch bei einem geringen Umsatz eine gute Produktausbeute erhalten werden kann.

Mit dem erfindungsgemäßen Verfahren gelingt es erstmals, Ketoisophoron mit Hilfe heterogener Katalysatoren in der Gasphase direkt zu TMCH zu hydrieren.

In den folgenden Beispielen werden als Begriffe verwendet:

Umsatz = (Mole umgesetzter Kohlenwasserstoff /

Mole eingesetzer Kohlenwasserstoff)

* 100 %

Ausbeute = (Mole erzeugtes Produkt / Mole

eingesetzer Kohlenwasserstoff) * 100 %

GHSV = Gas hourly space velocity
= (Volumen des eingespeisten Gases / Zeit x Schüttvolumen des Katalysators) [1/hl = 1/h]

Selektivität = (Ausbeute / Umsatz) * 100

Beispiele

Beispiel 1 (Aktivierung des Katalysators)

Der einzusetzende Katalysator wird im Stickstoffstrom im Reaktor auf eine Temperatur von 150°C mit einer Heizrate von 15°C pro Stunde geheizt, um dann mit Wasserstoff mit einer Heizrate von 10°C pro Stunde auf eine Temperatur von 240°C geheizt zu werden. Die Endtemperatur wird für 1 Stunde eingestellt. Die Temperatur im Katalysatorbett wird während der Aktivierung zur Kontrolle gemessen.

Beispiel 2

Es wird ein $Al_2O_3$ als Trägermaterial enthaltender Katalysator eingesetzt, der als aktive Bestandteile oxidische

Verbindungen von Na, Mg, Si, Ca, Co und metallisches Pd in einer Menge von jeweils > 0 bis < 1 Gew.-% und Cl in einer Menge von > 0 bis < 1 Gew.-% enthält.

Stündlich werden 924 1 Feedgas auf einen Liter Katalysator dem Reaktor zugeführt. Das Gasgemisch enthält je Mol KIP 6.5 Mol Wasserstoff und 2.86 Mol Stickstoff. Das Reaktorrohr wurde auf 240°C gehalten. Die Analyse des Produktgases erfolgt mittels Gaschromatograph. Es wurden 78 % des eingesetzten KIP umgesetzt. Die Ausbeute an THCM bezogen auf das eingesetzte KIP betrug 27 %.

Beispiel 3

Es wird ein Extrudat mit $Al_2O_3$ als Trägermaterial eingesetzt, das als aktive Bestandteile oxidische Verbindungen der Elemente Mg, Si, Ti, Cu und Zn und Cl und Sulfat in einer Menge von jeweils > 0 bis < 1 Gew.-% und NiO in einer Menge von ca. 17,9 Gew.-% enthält.

Stündlich werden 920 1 Feedgas auf einen Liter Katalysator dem Reaktor zugeführt. Das Gasgemisch enthält je Mol KIP 7.1 Mol Wasserstoff und 3.1 Mol Stickstoff. Der Katalysator wurde auf 258°C gehalten. Die Analyse des Produktgases erfolgt mittels on-line GC-MS. Es wurden 95 % des eingesetzten KIP umgesetzt. Die Ausbeute an THCM bezogen auf das eingesetzte KIP betrug 37 %.

Beispiel 4

Es wird ein Extrudat mit $Al_2O_3$ als Trägermaterial eingesetzt, das als aktive Bestandteile oxidische Verbindungen der Elemente Mg, Si, Ti, Cu und Zn und Cl und Sulfat in einer Menge von jeweils > 0 bis < 1 Gew.-% und NiO in einer Menge von ca. 18,15 Gew.-% enthält.

Stündlich werden 920 1 Feedgas mit einer Temperatur von 240°C auf einen Liter Katalysator dem Reaktor zugeführt. Das Gasgemisch enthält je Mol KIP 7.5 Mol Wasserstoff und 3.1 Mol Stickstoff. Der Katalysator wurde auf 250°C gehalten. Die Analyse des Produktgases erfolgt mittels on-line GC-MS. Es wurden 96 % des eingesetzten KIP umgesetzt. Die Ausbeute an THCM bezogen auf das eingesetzte KIP betrug 16 %.

**Patentansprüche**

1. Verfahren zur Herstellung von 2,6,6-Trimethyl-2-cyclohexan-1-dion (TMCH) durch partielle Hydrierung von 2,6,6-Trimethyl-2-cyclohexen-1,4-dion unter Verwendung von heterogenen Katalysatoren, **dadurch gekennzeichnet, dass** man die Reaktion in der Gasphase durchführt und als Katalysatoren Oxide oder Mischoxide von Elementen der Gruppe $I_a$, $II_a$, VIII, $I_b$, $II_b$, $III_a$, $IV_a$, $IV_b$, $VI_b$ und $V_a$ des Periodensystems der Elemente einzeln oder im Gemisch miteinander, wobei die Katalysatoren Pd oder Pt gegebenenfalls in metallischer Form enthalten.

2. Verfahren gemäss Anspruch 1, **dadurch gekennzeichnet, dass** man einen Trägerkatalysator einsetzt, der mindestens zwei zusätzliche oxidische Verbindungen in einer Menge von jeweils > 0 bis 2 Gew.-% enthält.

3. Verfahren gemäss Anspruch 2, **dadurch gekennzeichnet, dass** der eingesetzte Katalysator NiO in einer Menge von > 0,01 bis 50 Gew.-% enthält.

4. Verfahren gemäß Anspruch 1, **dadurch gekennzeichnet, dass** der eingesetzte Katalysator neben NiO eines oder mehrere der Oxide $TiO_2$, $SiO_2$, ZnO, $Fe_2O_3$, CuO oder MgO enthält.

5. Verfahren gemäß Anspruch 4, **dadurch gekennzeichnet, dass** der eingesetzte Katalysator NiO, CuO und ZnO enthält mit der Massgabe, dass NiO in einer Menge von 5 bis 25 Gew.-% und CuO und ZnO in einer Menge von jeweils > 0 bis 2 Gew.-% vorhanden sind.

6. Verfahren gemäß Anspruch 4, **dadurch gekennzeichnet, dass** der eingesetzte Katalsytor NiO, CuO und ZnO und gegebenenfalls Pt enthält mit der Massgabe, dass NiO in einer Menge von > 0 bis 2 Gew.-% und CuO und ZnO in einer Menge von zusammen 5 bis 40 Gew.-% vorhanden sind.

7. Verfahren gemäß Anspruch 1, **dadurch gekennzeichnet, dass** man Katalysatoren einsetzt, die oxidische Verbindungen der Elemente, ausgewählt aus der Gruppe Na, K, Mg, Ca, Sr, Ba, Ti, Zr, Mo, Mn, Fe, Co, Ni, Cu, Zn, Al, Si oder Sn enthalten, wobei mindestens zwei Verbindungen zusätzlich zum gegebenenfalls oxidischen Trägermaterial vorhanden sind.

8. Verfahren gemäß Anspruch 1, **dadurch gekennzeichnet, dass** man einen Katalysator einsetzt, der Kupferchromit in einer Menge von 40 bis 60 % und ZnO und $Fe_2O_3$ in einer Menge von jeweils 0,01 bis 1 Gew.-% enthält.

9. Verfahren gemäß Anspruch 1, **dadurch gekennzeichnet, dass** man einen Katalysator einsetzt, der Chlorid, Sulfat oder Phosphat, einzeln oder gemeinsam, in einer Menge von jeweils > 0 bis 5 Gew.-% enthält.

10. Verfahren gemäss Anspruch 1, **dadurch gekennzeichnet, dass** der eingesetzte Katalysator Pt oder Pd in einer Menge > 0 bis 1 Gew.-% auf $Al_2O_3$ oder $SiO_2$ als Trägermaterial und mindestens eine weitere oxidische Verbindung enthält.

11. Verfahren gemäß den Ansprüchen 1 bis 10, **dadurch gekennzeichnet, dass** man das Verfahren bei 180 bis 400°C, insbesondere 200 bis 280°C, durchführt.

12. Verfahren gemäß den Ansprüchen 1 bis 11, **dadurch gekennzeichnet, dass** man bei einem Druck von 1 bis 10 bara arbeitet.

13. Verfahren gemäß den Ansprüchen 1 bis 12, **dadurch gekennzeichnet, dass** je Liter Schüttvolumen des Katalysators 1 bis 10 Mol KIP/h eingespeist werden.

14. Verfahren gemäß den Ansprüchen 1 bis 13, **dadurch gekennzeichnet, dass** man das Verfahren kontinuierlich durchführt.

Europäisches Patentamt

# EUROPÄISCHER RECHERCHENBERICHT

Nummer der Anmeldung
EP 04 01 4734

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| D,A | M. VON ARX ET AL: "Unprecedented selectivity behaviour in the hydrogenation of an alpha,beta-unsaturated ketone: hydrogenation of ketoisophorone over alumina-supported Pt and Pd" J. MOL. CATALYSIS, Bd. 148, 1999, Seiten 275-283, XP002307224 * das ganze Dokument * ----- | 1-14 | C07C49/403 C07C45/62 |
| D,A | US 4 250 332 A (GRANDA EDWARD J ET AL) 10. Februar 1981 (1981-02-10) * Beispiel I * ----- | 1-14 | |
| A | DENNIS LIOTTA AND WALTER OTT: "Triene Cyclizations. The Total Synthesis of Pallescensin A." SYNTHETIC COMMUNICATIONS, Bd. 17, Nr. 14, 1987, Seiten 1655-1665, XP009040474 * Seite 1660, Zeilen 8-24 * ----- | 1-14 | |

RECHERCHIERTE SACHGEBIETE (Int.Cl.7)
C07C

Der vorliegende Recherchenbericht wurde für alle Patentansprüche erstellt

| Recherchenort | Abschlußdatum der Recherche | Prüfer |
|---|---|---|
| München | 29. November 2004 | Cooper, S |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 04 01 4734

*In diesem Anhang sind die Mitglieder der Patentfamilien der im obengenannten europäischen Recherchenbericht angeführten Patentdokumente angegeben.*
Die Angaben über die Familienmitglieder entsprechen dem Stand der Datei des Europäischen Patentamts am
Diese Angaben dienen nur zur Unterrichtung und erfolgen ohne Gewähr.

29-11-2004

| Im Recherchenbericht angeführtes Patentdokument | Datum der Veröffentlichung | Mitglied(er) der Patentfamilie | Datum der Veröffentlichung |
|---|---|---|---|
| US 4250332 A | 10-02-1981 | DE 3062773 D1 | 19-05-1983 |
| | | EP 0032287 A1 | 22-07-1981 |
| | | JP 1041623 B | 06-09-1989 |
| | | JP 1557152 C | 16-05-1990 |
| | | JP 56071033 A | 13-06-1981 |
| | | US 4289145 A | 15-09-1981 |
| | | US 4284654 A | 18-08-1981 |
| | | US 4501762 A | 26-02-1985 |

EPO FORM P0461

Für nähere Einzelheiten zu diesem Anhang : siehe Amtsblatt des Europäischen Patentamts, Nr.12/82